# EUROPEAN PATENT SPECIFICATION

(11) **EP 1 013 771 B1**
(45) Date of publication and mention of the grant of the patent: **06.10.2004**
(21) Application number: 99124650.5
(22) Date of filing: 24.02.1989
(51) Int. Cl.: C12N 15/86, C12N 5/14, A01H 5/00

(54) **Non-nuclear chromosomal transformation**
Nichtnukleare chromosomale Transformation
Transformation chromosomique non-nucléaire

(30) Priority: 26.02.1988 US 160771; 26.02.1988 US 160766; 17.02.1989 US 310881
(43) Date of publication of application: 28.06.2000
(62) Divisional of application: 89903418.5
(73) Proprietor: Large Scale Biology Corporation, Vacaville, CA 95688 (US)
(72) Inventor: Grill, Lawrence K., Los Altos, CA 94022 (US); Erwin, Robert L., Vacaville, CA 95687 (US); Berliner, David L., Atherton, CA 94025 (US); Hubbard, Ernest T.,Jr., Napa, CA 94558 (US); Turpen, Thomas H., Vacaville, CA 95687 (US)
(74) Representative: Viering, Jentschura & Partner

(56) References cited:
- EP-A- 0 067 553
- EP-A- 0 194 809

## Description

### BACKGROUND OF THE INVENTION

The present invention relates to viral vectors which are non-infective (also referred to herein as biologically contained) but which are self-replicating and capable of the non-nuclear chromosomal transformation of a host. The viral vectors may contain a heterologous coding sequence adjacent a viral promoter. The invention further relates to viruses containing the viral vectors which are transmissible. A host is infected by the viruses of the invention. Production cells are disclosed which are capable of producing the viruses or parts thereof.

Viruses are a unique class of infectious agents whose distinctive features are their simple organization and their mechanism of replication. In fact, a complete viral particle, or virion, may be regarded mainly as a block of genetic material (either DNA or RNA) capable of autonomous replication, surrounded by a protein coat and sometimes by an additional membranous envelope such as in the case of alpha viruses. The coat protects the virus from the environment and serves as a vehicle for transmission from one host cell to another.

Unlike cells, viruses do not grow in size and then divide, because they contain within their coats few or none of the biosynthetic enzymes and other machinery required for their replication. Rather, viruses multiply in cells by synthesis of their separate components, followed by assembly. Thus the viral nucleic acid, after shedding its coat, comes into contact with the appropriate cell machinery where it specifies the synthesis of proteins required for viral reproduction. The viral nucleic acid is then itself replicated through the use of both viral and cellular enzymes. The components of the viral coat are formed and the nucleic acid and coat components are finally assembled. With some viruses, replication is initiated by enzymes present in virions.

Viruses are subdivided into three main classes --animal viruses, plant viruses and bacterial viruses. Within each class, each virus is able to infect only certain species of cells. with animal and bacterial viruses, the host range is determined by the specificity of attachment to the cells which depends on properties of both the virion's coat and specific receptors on the cell surface. These limitations disappear when transfection occurs, i.e., when infection is carried out by the naked viral nucleic acid, whose entry does not depend on virus-specific receptors.

A given virus may contain either DNA or RNA, which may be either single- or double-stranded. The portion of nucleic acid in a virion varies from about 1% to about 50%. The amount of genetic information per virion varies from about 3 to 300 kb per strand. The diversity of virus-specific proteins varies accordingly. Examples of double-stranded DNA containing viruses include, but are not limited to, Hepatitis B virus, papovaviruses such as polyoma and papilloma, adenovirus, poxviruses such as vaccinia, caulimoviruses such as Cauliflower mosaic virus (CaMV), Pseudomonas phage PMS2, Herpesvirus, Bacillus subtilin phage SP8, and the T bacteriophages. Representative viruses which are single-stranded DNA are the parvoviruses and the bacteriophages φX174, fl and M13. Reoviruses, cytoplasmic polyhedrosis virus of silkworm, rice dwarf virus and wound tumor virus are examples of double-stranded RNA viruses. Single-stranded RNA viruses include tobacco mosaic virus (TMV), turnip yellow mosaic virus (TYMV), picornaviruses, myxoviruses, paramyxoviruses and rhabdoviruses. The RNA in single-stranded RNA viruses may be either a plus (+) or a minus (-) strand. For general information concerning viruses see Grierson, D. et al., Plant Molecular Biology, Blackie, London, pp. 126-146 (1984); Dulbecco, R. et al., Virology, Harper & Row, Philadelphia (1980); White, A. et al., Principles of Biochemistry, 6th Ed., McGraw-Hill, New York, pp. 882-900 (1978).

One means for classifying plant viruses is based on the genome organization. Although many plant viruses have RNA genomes, the organization of genetic information differs between groups. The genome of most monopartite plant RNA viruses is a single-stranded molecule of (+)- sense. There are at least 11 major groups of viruses with this type of genome. An example of this type of virus is TMV. At least six major groups of plant RNA viruses have a bipartite genome. In these, the genome usually consists of two distinct (+)-sense single-stranded RNA molecules that are encapsidated in separate particles. Both RNAs are required for infectivity. Cowpea mosaic virus (CPMW) is an example of a bipartite plant virus. The third major type, containing at least six major types of plant viruses, has three (+)-sense single-stranded RNA molecules, i.e., is tripartite. Each strand is separately encapsidated and all three are required for infectivity. An example of a tripartite plant virus is alfalfa mosaic virus (AMV). Many plant viruses also have smaller sub-genomic mRNAs that are synthesized to amplify a specific gene product. One group of plant viruses which have a single-stranded DNA genome are the geminiviruses, such as cassava latent virus and maize streak virus.

Techniques have been developed which are utilized to transform many species of organisms. Hosts which are capable of being transformed by these techniques include bacteria, yeast, fungus, animal cells and plant cells or tissue. Transformation is accomplished by using a vector which is self-replicating and which is compatible with the desired host. The vectors are generally based on either a plasmid or a virus. Foreign DNA is inserted into the vector, which is then used to transform the appropriate host. The transformed host is then identified by selection or screening. For further information concerning the transformation of these hosts, see Maniatis, T. et al., Molecular Cloning, Cold Spring Harbor Laboratory, Cold Spring Harbor (1982); DNA Cloning, Ed. Glover, D.M., IRL Press, Oxford (1985); Grierson, D. et al., supra; and Methods in Enzymology, volumes 68, 100, 101, 118 and 152-155 (1979, 1983, 1983, 1986 and 1987).

Viruses that have been shown to be useful for the transformation of appropriate hosts include bacteriophages, animal viruses such as adenovirus type 2 and vaccinia virus and plant viruses such as CaMV and brome mosaic virus (BMV). An example of the use of a bacteriophage vector is shown in U.S. Patent 4,508,826. U.S. Patent 4,593,002 shows the use of adenovirus type 2 as well as a bacteriophage for the transformation of the appropriate host. The use of a vaccinia virus is shown in U.S. Patent 4,603,112. Transformation of plants using plant viruses is described in EP A 67,553 (TMV), EP A 194,809 (BMV) and Brisson, N. et al., Methods in Enzymology 118, 659 (1986) (CaMV).

European Patent Application EP 0194809 discloses a RNA transformation vector comprising a *cis*-acting replication element derived of a (+)-strand RNA virus, such as brome mosaic virus, as well as a heterologous nucleic acid sequence, which is inserted into a region of the viral genome able to tolerate such insertion without disrupting viral replication. Thus, the modified RNA encoding the exogenous sequence can be expressed in the recipient cell.

International Patent Application WO 87/00551 discloses a retroviral vector for transmitting heterologous genetic material into the genome of plants. The vector comprises 5'-and 3'-retroviral long terminal repeat (LTR) sequences enabling insertion of retroviral sequences into a plant cell genome, a promoter sequence, and a nucleic acid sequence under the transcriptional control of said promoter that encodes a protein of interest imparting a new characteristic to the plant.

Finally, Takamatsu et al. (*EMBO J*. 6, 307-311, 1987) report the construction of tobacco mosaic virus (TMV) cDNA derivatives, wherein the viral coat protein gene is replaced by the bacterial chloramphenicol acteyltransferase gene (CAT). When *in vitro* transcripts of these TMV-CAT chimeras were inoculated in tobacco plants, TMV-specific lesions were produced. Northern blot analyses of plant RNA extracts revealed replication of the derivative genomic RNAs in the infected plant cells and production of subgenomic RNAs.

When the virus is a DNA virus, the constructions can be made to the virus itself. Alternatively, the virus can first be cloned into a bacterial plasmid for ease of constructing the desired viral vector with the foreign DNA. The virus can then be excised from the plasmid. If the virus is a DNA virus, a bacterial origin of replication can be attached to the viral DNA which is then replicated by the bacteria. Transcription and translation of this DNA will produce the coat protein which will encapsidate the viral DNA. If the virus is an RNA virus, the virus is generally cloned as a cDNA and inserted into a plasmid. The plasmid is then used to make all of the constructions. The RNA virus is then produced by transcribing the viral sequence of the plasmid and translation of the viral genes to produce the coat protein(s) which encapsidate the viral RNA.

Tyrosinase is a copper-containing monooxygenase catalyzing the ortho hydroxylation of monophenols, including tyrosine and the oxidation of o-diphenols to o-quinones. Mason, H.S., Ann. Rev. Biochem. 34, 595 (1965). Such reactions include the conversion of tyrosine to dihydroxyphenylalanine (dopa) and the conversion of dopa to dopaquinone. These reactions may be followed by the spontaneous formation of melanin. Tyrosinase occurs widely in nature and is responsible for the formation of melanin pigments. Lerch, K., Met.Ions Biol. Syst. 13, 143 (1981).

Tyrosinase is a phenoloxidase found specifically from mammalian sources. Other phenoloxidases or polyphenoloxidases may be found from plant or fungal sources. Phenoloxidases catalyze the oxidation of phenolic compounds (e.g., 3, 4 - dihydroxyphenylalanine (dopa)) to produce melanin (Wheeler, M.H. et al., Can. J. Microbiol. 24, 289 (1978)). A well-defined phenoloxidase is that found in the fungus Cryptococcus neoformans as described by Polachec, I. et al., J. Bacterio. 150, 1212 (1982). Another well-defined phenoloxidase is Drosophilia phenoloxidase described by Rizki, T.M. et al., Mol. Gen. Genet. 201, 7 (1985).

Cyclodextrin glucanotransferase is an enzyme catalyzing the conversion of starch to cyclodextrin. Cyclodextrin has several uses including use as a sweetening substance, use as a substitute for gum arabic and other uses in medicines, foods and cosmetics.

### SUMMARY OF THE INVENTION

The present invention is based on viral vectors as defined in claim 1 which are biologically contained, self-replicating and capable of the non-nuclear chromosomal transformation of a host. The viral vectors may contain a heterologous coding sequence adjacent a viral promoter.

The invention relates to production cells as defined in claim 1 which are capable of producing the viruses or parts thereof. Host cells are infected by the viruses used in the invention. A process for the production of a desired product by growing an infected plant cell is also within the scope of the present invention.

### DETAILED DESCRIPTION OF THE INVENTION

The present invention includes (a) viral vectors which are non-infective but which are self-replicating and capable of the non-nuclear chromosomal transformation of a host, (b) viral vectors which contain a heterologous coding sequence adjacent a viral promoter, (c) viruses containing the viral vectors which are infective, (d) production cells which are capable of producing the viruses or parts thereof, (e) a plant cell infected by the viruses used in the invention, and (f) a process for the production of a desired product by growing an infected plant cell.

In order to provide a clear and consistent understanding of the specification and the claims, including the scope given to such terms, the following definitions are provided:
Adjacent: A position in a nucleotide sequence immediately 5' or 3' to a defined sequence.
Anti-sense Mechanism: A type of gene regulation based on controlling the rate of translation of mRNA to protein due to the presence in a cell of an RNA molecule complementary to at least a portion of the mRNA being translated.
Biologically Contained: The viral nucleic acid is not capable of naturally infecting a host since it is not capable of expressing a biologically functional coat protein.
Biologically Functional: The capability of performing an expected biological function in a cell or organism. For example, the biological function of a viral coat protein is the encapsidation of the viral nucleic acid. A non-biologically functional coat protein is not capable of encapsidating viral nucleic acid. A nucleotide sequence which lacks a biologically functional protein coding sequence will produce either no protein or will produce a protein which will not perform an expected function. If the entire coding sequence for the protein is removed, then no protein will be produced. If a significant portion of the coding sequence for the protein is removed, then any protein that is produced will not function as the entire protein would function. If the coding sequence for the protein is mutated such as by a point mutation, the protein might not function as a normal protein would function. For example, a nucleotide sequence which lacks a biologically functional coat protein coding sequence is a nucleotide sequence which does not code for a coat protein capable of encapsidating viral nucleic acid. This term is intended to include a complete deletion of the coat protein sequence.
Cell Culture: A proliferating mass of cells which may be in an undifferentiated or differentiated state.
Chimeric Sequence or Gene: A nucleotide sequence derived from at least two heterologous parts. The sequence may comprise DNA or RNA.
Coding Sequence: A deoxyribonucleotide sequence which when transcribed and translated results in the formation of a cellular polypeptide, or a ribonucleotide sequence which when translated results in the formation of a cellular polypeptide.
Compatible: The capability of operating with other components of a system. A vector which is compatible with a host is one which is capable of replicating in that host. A coat protein which is compatible with a viral nucleotide sequence is one which is capable of encapsidating the viral sequence.
Gene: A discrete chromosomal region which is responsible for a discrete cellular product.
Host: A cell, tissue or organism capable of replicating a viral vector and which is capable of being infected by a virus containing the viral vector. This term is intended to include eukaryotic cells, organs, tissues or organisms, such as plant tissue.
Infection: The ability of a virus to transfer its nucleic acid to a host wherein the viral nucleic acid is replicated, viral proteins are synthesized and new viral particles assembled. The terms transmissible and infective are used interchangeably herein. The term non-infective as used herein means non-infective by natural, biological means.
Phenotypic Trait: An observable property resulting from the expression of a gene.
Plant Cell: The structural and physiological unit of plants, consisting of a protoplast and the cell wall.
Plant Organ: A distinct and visibly differentiated part of a plant such as root, stem, leaf or embryo.
Plant Tissue: Any tissue of a plant in plant or in culture. This term is intended to include a whole plant, plant cell, plant organ, protoplast, cell culture or any group of plant cells organized into a structural and functional unit.
Production Cell: A cell, tissue or organism capable of replicating a vector or a viral vector, but which is not necessarily a host to the virus. This term is intended to include prokaryotic and eukaryotic cells, organs, tissues or organisms, such as bacteria, yeast, fungus, animal cells and plant tissue.
Promoter: The 5'-flanking, non-coding sequence adjacent a coding sequence which is involved in the initiation of transcription of the coding sequence.
Protoplast: An isolated plant cell without cell walls, having the potency for regeneration into cell culture or a whole plant.
Substantial Sequence Homology: Denotes nucleotide sequences that are substantially equivalent to one another. Nucleotide differences between such sequences having substantial sequence homology will be de minimis in affecting the function of the gene products or an RNA coded for by such sequence.
Transcription: The production of an RNA molecule by RNA polymerase as a complementary copy of a DNA sequence.
Vector: A self-replicating DNA molecule which transfers a DNA segment between cells.
Viral Vector: A vector comprising a nucleic acid sequence of a virus which has been modified so that a non-biologically functional coat protein is produced. This may be accomplished by removing at least a part of the coding sequence or by mutating the coding sequence. If the virus codes for one or more virus transmissibility factors, then the nucleic acid sequence of the virus is also modified to make these non-biologically functional.
Virus: An infectious agent which is composed of a nucleic acid encapsidated in a protein. A virus may be a mono-, di-, tri- or multi-partite virus as described above.

The present invention provides for the infection of a host, such as a eukaryotic organism, cell or tissue, by a virus as defined in claim 1 which has been modified so that the virus is transmissible but the viral nucleic acid is not infective. Naturally occurring mutant viruses may also have these same properties of being transmissible, but having viral nucleic acid which is not infective. The non-infectivity of the viral nucleic acid is accomplished by modifying the nucleic acid so that biologically non-functional viral coat protein (capsid protein) and any other viral transmissibility factors are produced as described herein.

The present invention has a number of advantages, one of which is that the transformation and regeneration of target organisms is not necessary. Another advantage is that it is not necessary to develop vectors which integrate a desired coding sequence in the genome of the target organism. Existing organisms can be altered with a new coding sequence without the need of going through a germ cell. The present invention also gives the option of applying the coding sequence to the desired organism, tissue, organ or cell.

The chimeric genes and vectors of the present invention are constructed using techniques well known in the art. Suitable techniques have been described in Maniatis, T. et al., Molecular Cloning, Cold Spring Harbor Laboratory, New York (1982); Methods in Enzymology, Vols. 68, 100, 101, 118 and 152-155, Academic Press, New York (1979, 1983, 1983, 1986 and 1987); and DNA Cloning, Vols. I, II, III, Glover, D.M., Ed., IRL Press, Oxford (1985 and 1987). Medium compositions have been described in Miller, J.H., Experiments in Molecular Genetics, Cold Spring Harbor Laboratory, New York (1972), as well as the references previously identified. DNA manipulations and enzyme treatments are carried out in accordance with the manufacturers' recommended procedures.

An important feature of the present invention is the preparation of nucleotide sequences which are capable of replication in a compatible host but which in themselves are incapable of infecting the host. The nucleotide sequence has substantial sequence homology to a viral nucleotide sequence. Suitable viral nucleotide sequences are those of tobamo viruses which infect plants. A partial listing of suitable viruses has been described above. The nucleotide sequence may be an RNA, DNA, cDNA or chemically synthesized RNA or DNA.

The first step in achieving any of the features of the invention is to modify the nucleotide sequences coding for the capsid protein and any transmissibility factors within the viral nucleotide sequence by known conventional techniques such that non-biologically functional proteins are produced by the modified virus. Therefore, any virus as defined in claim 1 for which the capsid protein nucleotide sequence and any transmissibility factor nucleotide sequences have been identified may be suitable for use in the present invention. Other viruses may be used after the nucleic acid has been sequenced.

The viruses which meet this requirement, and therefore are suitable, are the viruses from the tobacco mosaic virus group such as Tobacco Mosaic virus (TMV), Cowpea Mosaic virus (CMV), Alfalfa Mosaic virus (AmV), Cucumber Green Mottle Mosaic virus watermelon strain (CGMMV-W) and Oat Mosaic virus (OMV).

### TOBACCO MOSAIC VIRUS GROUP

Tobacco Mosaic virus (TMV) is a type member of the Tobamoviruses. The TMV virion is a tubular filament, and comprises coat protein subunits arranged in a single right-handed helix with the single-stranded RNA intercalated between the turns of the helix. TMV infects tobacco as well as other plants. TMV is transmitted mechanically and may remain infective for a year or more in soil or dried leaf tissue.

The TMV virions may be inactivated by subjection to an environment with a pH less than 3 or greater than 8, or by formaldehyde or iodine. Preparations of TMV may be obtained from plant tissues by (NH₄)₂SO₄ precipitation followed by differential centrifugation.

The TMV single-stranded RNA genome is about 6400 nucleotides long and is capped at the 5' end but is not poly-adenylated. The genomic RNA can serve as mRNA for a protein of molecular weight about 130,000 (130K) and another produced by read-through of molecular weight about 180,000 (180K). However, it cannot function as a messenger for the synthesis of coat protein. Other genes are expressed during infection by the formation of monocistronic, 3'-coterminal subgenomic mRNAs, including one (LMC) encoding the 17.5K coat protein and another (I₂) encoding a 30K protein. The 30K protein has been detected in infected protoplasts (Virology 132, 71 (1984)), and it is involved in the cell-to-cell transport of the virus in an infected plant (Deom, C.M. et al., Science 237, 389 (1987)). The functions of the two large proteins are unknown.

Several double-stranded RNA molecules, including double-stranded RNAs corresponding to the genomic, I₂ and LMC RNAs, have been detected in plant tissues infected with TMV. These RNA molecules are presumably intermediates in genome replication and/or mRNA synthesis - processes which appear to occur by different mechanisms.

TMV assembly apparently occurs in the plant cell cytoplasm, although it has been suggested that some TMV assembly may occur in chloroplasts since transcripts of ctDNA have been detected in purified TMV virions. Initiation of TMV assembly occurs by interaction between ring-shaped aggregates ("discs") of coat protein (each disc consisting of two layers of 17 sub-units) and a unique internal nucleation site in the RNA; a hairpin region about 900 nucleotides from the 3' end in the common strain of TMV. Any RNA, including subgenomic RNAs, containing this site may be packaged into virions. The discs apparently assume a helical form on interaction with the RNA, and assembly (elongation) then proceeds in both directions (but much more rapidly in the 3'- to 5' direction from the nucleation site).

Another member of the Tobamoviruses, the Cucumber green mottle mosaic virus watermelon strain (CGMMV-W) is related to the cucumber virus. Noru, Y., et al., Virology 45, 577 (1971). The coat protein of CGMMV-W interacts with the RNA of both TMV and CGMMV to assemble viral particles in vitro. Kurisu et al., Virology 70, 214 (1976).

Several strains of the tobamovirus group are divided into two subgroups on the basis of the location of the assembly of origin. Fukuda, M. et al., Proc. Natl. Acad. Sci. USA 78, 4231 (1981). Subgroup I, which includes the vulgare, OM, and tomato strain, has an origin of assembly at about 800-1000 nucleotides from the 3' end of the RNA genome, and outside of the coat protein cistron. Lebeurier, G. et al., Proc. Natl. Acad. Sci. USA 74, 1913 (1977) ; and Fukuda, M. et al., Virology 101, 493 (1980). Subgroup II, which includes CGMMV-W and cornpea strain (Cc), has an origin of assembly about 300-500 nucleotides from the 3' end of the RNA genome, and within the coat-protein cistron. Fukuda, M. et. al., supra. The coat protein cistron of CGMMV-W is located at nucleotides 176-661 from the 3'end. The 3' noncoding region is 175 nucleotides long. The origin of assembly is positioned within the coat protein cistron. Meshi, T. et al., Virology 127, 52 (1983).

The nucleotide sequence of any suitable virus as defined in claim 1 can be derived from a viral nucleic acid by modifying the coat protein coding sequence. The modification may be the removal of a coding sequence for at least a part of the viral coat protein. Alternatively, the nucleotide sequence can be synthesized such that it lacks at least a part of the viral coat protein coding sequence. A sufficient amount of the coding sequence is removed such that any coat protein which may be produced by the virus will be incapable of encapsidating the viral nucleic acid. In addition, the coat protein coding sequence may be modified by mutation such that the coat protein which is produced is incapable of encapsidating the viral nucleic acid. In each instance, a non-biologically functional protein is produced. In some instances, part of the gene may be necessary for good promoter activity. If so, then the entire gene should not be deleted. In order to be easily transmissible to other plants, the nucleotide sequence must be encapsidated in a compatible coat protein as described further below. The viral nucleic acid may further be modified to alter the coding sequence for any viral transmissibility factors. The alteration of the coding sequences for these factors will ensure that the nucleotide sequence cannot be transmitted by other vectors, e.g. by insects.

The nucleotide sequence is prepared by cloning the viral nucleic acid in an appropriate production cell. If the viral nucleic acid is DNA, it can be cloned directly into a suitable vector using conventional techniques. One technique is to attach an origin of replication compatible with the production cell to the viral DNA. If the viral nucleic acid is RNA, a full-length DNA copy of the viral genome is first prepared by well known procedures. For example, the viral RNA is transcribed into DNA using reverse transcriptase to produce subgenomic DNA pieces, and a double-stranded DNA made using DNA polymerases. The DNA is then cloned into appropriate vectors and cloned into a production cell. The DNA pieces are mapped and combined in proper sequence to produce a full-length DNA copy of the viral RNA genome. The coding sequences for the viral coat protein and any viral transmissibility factors are identified and altered. The resulting nucleotide sequence is self-replicating but incapable of infecting host itself. Any manner of separating the coding sequences for the viral coat protein and the coding sequences for the viral transmissibility factors from the remainder of the viral nucleotide sequence or of altering the nucleotide sequences of these proteins to produce non-biologically functional proteins is suitable for the present invention.

In the case of alphaviruses, the E1 and E2 glycoproteins may play a role in transmissibility of the virus (Garaff, H. et al., Nature 288, 236 (1980)). These glycoproteins are incorporated in a liquid envelope which surrounds the coat protein. The nucleotide sequence which codes for the E1 and E2 glycoproteins is adjacent to the coding sequence for the coat protein in alphavirus RNA. Therefore the E1 and E2 glycoprotein coding sequences can be removed with the coat protein coding sequence by known conventional techniques.

A second feature of the present invention is a chimeric nucleotide sequence which comprises a first nucleotide sequence and a second nucleotide sequence. The first sequence is capable of self-replication, is not capable of transmission and has substantial sequence homology to a viral nucleotide sequence, as described above. The second sequence is capable of being transcribed in a host. The second sequence is preferably placed adjacent a viral promoter, although a fusion protein may be produced which also has biological activity. Any viral promoter can be utilized, but it is preferred to use a promoter of the viral coat protein gene, at least a part of the coding sequence of which has been deleted. In those instances where the coat protein coding sequence is altered but not deleted, a viral promoter can be attached to the second sequence by conventional techniques or the second sequence can be inserted into or adjacent the coat protein coding sequence such that a fusion protein is produced. The second sequence which is transcribed may be transcribed as an RNA which is capable of regulating the expression of a phenotypic trait by an anti-sense mechanism. Alternatively, the second sequence in the chimeric nucleotide sequence may be transcribed and translated in the host, e.g., plant tissue, to produce a phenotypic trait. The second nucleotide sequence may also code for the expression of more than one phenotypic trait. The chimeric nucleotide sequence is constructed using conventional techniques such that the second nucleotide sequence is in proper orientation to the viral promoter.

Useful phenotypic traits in plant cells include, improved tolerance to herbicides, improved tolerance to extremes of heat or cold, drought, salinity or osmotic stress; improved resistance to pests (insects, nematodes or arachnids) or diseases (fungal, bacterial or viral); production of enzymes or secondary metabolites; male or female sterility; dwarfness; early maturity; improved yield, vigor, heterosis, nutritional qualities, flavor or processing properties, and the like. Other examples include the production of important proteins or other products for commercial use, such as lipase, melanin, pigments, antibiotics and the like. Another useful phenotypic trait is the production of degradative or inhibitory enzymes, such as are utilized to prevent or inhibit root development in malting barley. The phenotypic trait may also be a secondary metabolite whose production is desired in a bioreactor.

An example of a chimeric nucleotide sequence is one which contains a first nucleotide sequence having substantial sequence homology to TMV and a second nucleotide sequence which is a coding sequence for tyrosinase. In a second example, the virus is oat mosaic virus (OMV). OMV is capable of infecting most monocot species including barley and corn. In a third example, the. second nucleotide sequence is the coding sequence for cyclodextrin glucanotransferase.

The second nucleotide sequence can be inserted into the first nucleotide sequence prepared above such that it is adjacent a viral promoter. Since the location of the promoter of the viral coat protein gene is known in this sequence as a result of the deletion of the gene, the second nucleotide sequence can be placed adjacent this promoter. Alternatively, an appropriate viral promoter can first be attached to the second nucleotide sequence and this construct can then be inserted either into the first nucleotide sequence or adjacent thereto. In addition, the second nucleotide sequence can be inserted into and adjacent an altered coat protein coding sequence.

A double-stranded DNA of the chimeric nucleotide sequence or of a complementary copy of the chimeric nucleotide sequence is cloned into a production cell. If the viral nucleic acid is an RNA molecule, the chimeric nucleotide sequence is first attached to a promoter which is compatible with the production cell. The chimeric nucleotide sequence can then be cloned into any suitable vector which is compatible with the production cell. In this manner, only RNA copies of the chimeric nucleotide sequence are produced in the production cell. For example, if the production cell is E. coli, the lac promoter can be utilized. If the production cell is a plant cell, the CaMV promoter can be used. The production cell will be a eukaryotic cell such as yeast, plant or animal, if viral RNA must be capped for biological activity. The chimeric nucleotide sequence can then be cloned into any suitable vector which is compatible with the production cell. Alternatively, the chimeric nucleotide sequence is inserted in a vector adjacent a promoter which is compatible with the production cell. If the viral nucleic acid is a DNA molecule, it can be cloned directly into a production cell by attaching it to an origin of replication which is compatible with the production cell. In this manner, DNA copies of the chimeric nucleotide sequence are produced in the production cell.

A promoter is a DNA sequence that directs RNA polymers to bind to DNA and to initiate RNA synthesis. There are strong promoters and weak promoters. Among the strong promoters are lacuv5, trp, tac, trp-lacuv5, λp₁, ompF, and bla. A useful promoter for expressing foreign genes in E. coli is both strong and regulated. The λp₁ promoter of bacteriophage λ is a strong, well-regulated promoter. Hedgpeth, J.M. et al. Mol. Gen. Genet. 163, 197 (1978); Bernard, H. M. et al. Gene 5, 59 (1979); Remaut, E.P. et al., Gene 15, 81 (1981).

A gene encoding a temperature-sensitive λ repressor such as λ*cIts* 857 may be included in the cloning vector. Bernard et al., supra. At low temperature (31°C), the p₁ promoter is maintained in a repressed state by the cI-gene product. Raising the temperature destroys the activity of the repressor. The p₁ promoter then directs the synthesis of large quantities of mRNA. In this way, E. coli production cells may grow to the desired concentration before producing the products encoded within the vectors. Similarly, a temperature-sensitive promoter may be activated at the desired time by adjusting the temperature of the culture.

It may be advantageous to assemble a plasmid that can conditionally attain very high copy numbers. For example, the pAS2 plasmid containing a lac or tac promoter will achieve very high copy numbers at 42°C. The lac repressor, present in the pAS2 plasmid is then inactivated by isopropyl-β-D-thiogalactoside to allow synthesis of mRNA.

A further alternative when creating the chimeric nucleotide sequence, is to prepare more than one nucleotide sequence (i.e., prepare the nucleotide sequences necessary for a multipartite viral vector construct). In this case, each nucleotide sequence would require its own origin of assembly. Each nucleotide sequence could be chimeric (i.e., each nucleotide sequence has a foreign coding sequence inserted therein), or only one of the nucleotide sequences may be chimeric.

If a multipartite virus were found to have the coding sequence for its coat protein on one strand of nucleic acid, and the coding sequence for a transmissibility factor on a different strand, then two chimeric nucleotide strands would be created in accordance with the invention. One foreign coding sequence would be inserted in place of the coat protein gene (or inserted next to the altered coat protein gene) on one strand of nucleic acid, and another foreign coding sequence would be inserted in place of the transmissibility factor gene (or inserted next to the altered transmissibility factor gene) on the other strand of nucleic acid.

Alternatively, the insertion of a foreign coding sequence into the nucleotide sequence of a monopartite virus may result in the creation of two nucleotide sequences (i.e., the nucleic acid necessary for the creation of a bipartite viral vector). This would be an advantageous situation when it is desirable to keep the replication and translation of the foreign coding sequence separate from the replication and translation of some of the coding sequences of the original nucleotide sequence. Each nucleotide sequence would have to have its own origin of assembly.

A third feature of the present invention is a virus or viral particle. The virus comprises a chimeric nucleotide sequence as described above which has been encapsidated. The resulting product is then capable of infecting an appropriate host, but the chimeric nucleotide sequence is incapable of further infection since it lacks the mechanism to produce additional virus or viral particles. However, the chimeric nucleotide sequence is capable of replicating in the host. The chimeric nucleotide sequence is transcribed and/or translated within the host to produce the desired product.

Most viruses are encapsidated by either an icosahedral capsid, a spherical capsid or a rod-shaped capsid. Plant viruses such as the tobacco mosaic virus are commonly encapsidated by a rod-shaped capsid.

The icosahedral capsids and the spherical capsids are more geometrically constrained than a rod-shaped capsid, and therefore are more limited as to the amount of nucleic acid which may be encapsidated. In contrast, a rod-shaped capsid is expandable so that it can encapsidate any amount of nucleic acid as explained in European Patent Application 0 278 667.

In one embodiment of the present invention, the chimeric nucleotide sequence is encapsidated by a heterologous capsid. Most commonly, this embodiment will make use of a rod-shaped capsid because of its ability to encapsidate a longer chimeric nucleotide sequence than the more geometrically constrained icosahedral capsid or spherical capsid. The use of a rod-shaped capsid permits the incorporation of a larger foreign protein coding sequence to form the chimeric nucleotide sequence. Such a rod-shaped capsid is most advantageous when more than one foreign coding sequence is present in the chimeric nucleotide sequence.

Another feature of the invention is a vector containing the chimeric nucleotide sequence as described above. The chimeric nucleotide sequence is adjacent a nucleotide sequence selected from the group consisting of a production cell promoter or an origin of replication compatible with the production cell. The vector is utilized to transform a production cell which will then produce the chimeric nucleotide sequence in quantity. The production cell may be any cell which is compatible with the vector. The production cell may be prokaryotic or eukaryotic. However, if the viral RNA (chimeric nucleotide sequence) must be capped in order to be active, then the production cell must be capable of capping the viral RNA, such as a eukaryotic production cell. The production cell may contain only the vector which contains the chimeric nucleotide sequence. In this instance, the production cell will only produce the chimeric nucleotide sequence which must then be encapsidated in vitro to produce an infective virus. The encapsidation is accomplished by adding a compatible viral coat protein to the chimeric nucleotide sequence in accordance with conventional techniques. In this scenario, the viral coat protein would be produced in a second production cell. A vector comprising a coding sequence for a compatible viral coat protein adjacent a promoter compatible with the production cell is utilized to transform the second production cell. The second production cell then produces the viral coat protein in quantity.

As explained above, the viral coat protein produced by the second production cell may be homologous or heterologous to the viral nucleotide sequence. The viral coat protein must be compatible with the chimeric nucleotide sequence and of sufficient size to completely encapsidate the chimeric nucleotide sequence.

Alternatively, the production cell has a coding sequence for a compatible viral coat protein adjacent a promoter compatible with the production cell stably incorporated into its genome. Again, this coding sequence for the viral coat protein may be homologous or heterologous to the viral nucleotide sequence. In this instance, the production cell then produces the coat protein and the chimeric nucleotide sequence. The chimeric nucleotide sequence is then encapsidated by the viral coat protein to produce a transmissible virus.

With most viruses, it is unknown whether transmissibility factors alone are essential for the infectivity of a virion or whether the transmissibility factors simply serve as an adjunct to the capsid, and therefore enhance virion infectivity, but do not prevent infection if not present on the virion. For purposes of this invention (i.e., to insure non-infectiousness of the viral vector), it has been assumed that the transmissibility factors alone could render the viral vector infectious, and therefore the vector comprising the chimeric nucleotide sequence will contain neither a nucleotide sequence for the capsid protein nor a nucleotide sequence for any transmissibility factor.

However, this vector comprising the chimeric nucleotide sequence must contain a promoter and an origin of replication, and preferably contains an origin of assembly. As stated above, this vector is utilized to transform a production cell. The origin of replication must be compatible with the production cell.

All of the promoters associated with the vector must also be compatible with the production cell in which the vector is utilized. The prokaryotic production cell or a eukaryotic production cell is transformed such that the coding sequences for the coat protein and transmissibility factors (if necessary) are stably incorporated into the genome of the cell. Several conventional techniques can be utilized to accomplish this embodiment. For example, appropriate coding sequences can be inserted into plant cells by transformation with Agrobacterium, such as described by Schell, J. et al., Bio/Technology 1, 175 (1983); Fillatti, J. et al., Bio/Technology 5, 726 (1987); Everett, N.P. et al., Bio/Technology 5, 1201 (1987); Pua, E-C., Bio/Technology 5, 815 (1987); Hinchee, M.A., et al., Bio/Technology 6, 915 (1988) and Meth. Enzymol, Vol. 118, supra. Agrobacterium have also been utilized for introducing viruses into plants, both dicots and monocots by a process termed Agro-infection. This technique has been described by Grimsley, N., et al., Proc. Natl. Acad. Sci, USA 83, 3282 (1986); Elmer, J.S. et al., Plant Mol Biol 10, 225 (1988); Grimsley, N. et al., Nature 325, 177 (1987); and Hayes, R.J. et al., Nature 334, 180 (1988).

Alternatively, the appropriate coding sequence can be inserted into eukaryotic cells by direct gene transfer including electroporation, calcium chloride or polyethylene glycol mediated transformation, liposome fusion microinjection or microprojectile bombardment. These techniques have been described by Fromm, M.E., Meth. Enzymol 153, 307 (1987); Shillito, R.D. et al., Meth. Enzymol 153, 283 (1987); Dehayes, A., et al., EMBO J 4, 2731 (1985); Negrutin, R. et al., Plant Mol Biol 8, 363 (1987); Reich, T.J. et al., Bio/Technology 4, 1001 (1986); Klein, T.M. et al., Bio/Technology 6, 559 (1988); McCabe, D.E. et al., Bio/Technology 6, 923 (1988).

A production cell which has been transformed will contain the coat protein and/or transmissibility factors coding sequence(s) stably incorporated in the genome. The vector containing the chimeric nucleotide sequence is then introduced into the production cell as previously discussed.

Once the chimeric nucleotide sequence is replicated and the capsid protein and transmissibility factors have been produced by the appropriate production cells, the intact virions may be assembled in vitro. The replicated chimeric nucleotide sequence is encapsidated by the capsid protein in accordance with known conventional techniques. The transmissibility factors may be added to the virion assembly separately.

A further feature of the present invention is a host which has been infected by the virus. After introduction into a host, the host contains the chimeric nucleotide sequence which is capable of self-replication but which is not capable of producing additional transmissible viruses or viral particles. The host can be infected with the virus by conventional techniques. Suitable techniques include leaf abrasion, abrasion in solution, high velocity water spray and other injury of a host as well as imbibing host seeds with water containing the chimeric nucleotide sequence alone or the encapsidated virus. Although the chimeric nucleotide sequence is not capable of producing infective agents, it is capable of self-replicating and spreading throughout the plant host.

An alternative method for introducing a chimeric nucleotide sequence into a plant host is a technique known as agroinfection or Agrobacterium-mediated transformation (sometimes called Agro-infection) as described by Grimsley, N. et al., Nature, 325, 177 (1987). This technique makes use of a common feature of Agrobacterium which colonize plants by transferring a portion of their DNA (the T-DNA) into a host cell, where it becomes integrated into nuclear DNA. The T-DNA is defined by border sequences which are 25 base pairs long, and any DNA between these border sequences is transferred to the plant cells as well. The insertion of a chimeric viral nucleotide sequence between the T-DNA border sequences results in transfer of the chimeric sequence to the plant cells, where the chimeric sequence is replicated, and then spreads systemically through the plant. Agro-infection has been accomplished with potato spindle tuber viroid (PSTV) (Gardner, R.C. et al., Plant Mol. Biol. 6, 221 (1986)), cauliflower mosaic virus (CaMV) (Grimsley, N. et al., Proc. Natl. Acad. Sci. U.S.A. 83, 3282 (1986)), maize streak virus (Grimsley, N. et al., Nature 325, 177 (1987) and Lazarowitz, S.G., Nucl. Acids Res. 16, 229 (1988)), digitaria streak virus (Donson, J. et al., Virogology 162, 248 (1988)), wheat dwarf virus (Hayes, R.J. et al., J. Gen. Virol. 69, 891 (1988)) and tomato golden mosaic virus (TGMV) (Elmer, J.S. et al., Plant Mol. Biol. 10, 225 (1988) and Gardiner, W.E. et al., EMBO J. 7, 899 (1988)). Therefore, agro-infection of a susceptible plant could be accomplished with a virion containing a chimeric nucleotide sequence based on the nucleotide sequence of any of the above viruses.

Several viral products are useful to insure the production and spread of viral nucleic acids. One factor is a replicase which is involved in the replication of the viral nucleic acid. A second factor which may be present is termed herein a transport protein. This protein(s) is(are) involved in the movement of the viral nucleic acid from infected cells to adjacent cells and thus, the spread of the viral nucleic acid throughout the production or host cell, tissue or organism. In order to insure suitable replication and spread of the viral vector, an additional embodiment of the present invention includes the stable transformation of the host or production cell, tissue or organism with a coding sequence for viral replicase, a viral transport protein or both. The transformation, including stable integration into the genome of the production cell or host, is accomplished as described above concerning the coat protein gene.

In an additional embodiment of the invention, the host range of a virus is enlarged so that a viral construct, i.e., chimeric nucleic acid, containing different foreign genes may be used in many different hosts. The host range of the virus is enlarged by transforming the host (or production cell since it will also work in this context) to contain parts of viral material useful in enabling infection of the host by the viral construct. For plant hosts, the hosts are transformed to contain the viral replicase gene or transport protein gene. Alternatively, the virus can be produced to contain coding sequences for transport proteins capable of functioning in the host. In addition, it is possible to transform protoplasts of the host plant to contain the viral nucleic acid or parts thereof such that all cells of the regenerated plants will be capable of infection. This transformation is carried out by any of the techniques previously described.

A still further feature of the invention is a process for the production of a specified product such as an anti-sense RNA, a ribozyme, a protein, an enzyme or a complex biomolecule. Such products include those discussed above. The second nucleotide sequence of the chimeric nucleotide sequence comprises the transcribable sequence which leads to the production of the desired product. This process involves the infection of the appropriate host with a virus, such as those described above, the growth of the infected host to produce the desired product and the isolation of the desired product, if necessary. The growth of the infected host is in accordance with conventional techniques, as is the isolation of the resultant product.

In one example, a tyrosinase coding sequence such as isolated from Streptomyces antibioticus is inserted adjacent the promoter of the viral coat protein of a nucleotide sequence derived from TMV oat mosaic virus (OMV) in which the coat protein coding sequence has been removed. A virus is prepared as described above using the resulting chimeric nucleotide sequence. Tobacco or germinating barley is infected with the appropriate virus. The chimeric nucleotide sequence self-replicates in the plant tissue to produce the enzyme tyrosinase. Alternatively, the tyrosinase coding sequence is inserted into other appropriately modified viral nucleic acid. The resultant virus is used to infect the appropriate host and tyrosinase is produced. The activity of this enzyme leads to the production of melanin. See for example, Huber, M. et al., Biochemistry 24, 6038 (1985).

In a second example, a cyclodextrin glucanotransferase coding sequence, such as isolated from Bacillus sp. No. 17-1 (see U.S. Patent 4,135,977) is inserted adjacent the promoter of the viral coat protein of a nucleotide sequence derived from OMV, in which the coat protein coding sequence has been removed. A virus is prepared as described above using the resulting chimeric nucleotide sequence. Corn or potato is infected with the appropriate virus. The chimeric nucleotide sequence self-replicates in the plant tissue to produce the enzyme cyclodextrin glucotransferase. The activity of this enzyme leads to the production of cyclodextrin, which is useful as flavorant or for drug delivery.

### EXAMPLES

The following examples further illustrate the present invention. In these examples, enzyme reactions were conducted in accordance with the manufacturer's recommended procedures unless otherwise indicated. Standard techniques such as those described in Maniatis, T. et al., supra; Meth. in Enzymol, Vol. 68, 100, 101, 118, 152-155, supra; and DNA Cloning, Vol. I, II, III, supra, were utilized for vector constructions and transformation unless otherwise specified.

### EXAMPLE 1

### Preparation of Chimeric TMV DNA Comprising Tyrosinase Gene in Place of the Coat Protein Gene

The 1.2 kb tyrosinase gene was derived from the pIJ702 streptomyces plasmid (available to the public) using a SacI/PVUII cut. It was inserted into the SacI/EcoRV site of the Stratagene bluescript vector (KS), which is 2.9 kb in size. The resulting plasmid was maintained as pBG110 (4.1 kb). The tyrosinase gene was removed from pBG110 by a SacI/HindIII cut and inserted into pUC19 (2.7 kb) giving pBG115 (3.9 kb). The tyrosinase gene was removed from pBG115 by a EcoRI/HindIII cut and inserted into the Stratagene bluescript vector (KS+; 2.9 kb) giving pBG120 (4.1 kb). The tyrosinase gene was removed from pBG120 with a XhoI/Smal cut and XhoI linkers were added to the SmaI end. This tyrosinase gene with XhoI ends was inserted into the XhoI site of the Stratagene bluescript vector (KS+; 2.9 kb) to give pBG130 (4.1 kb). Since there is an unwanted PstI site carried over in a poly-linker region, the pBG130 was modified by removing some of the non-coding bases and the poly-linker region. This was performed using a HindIII/SacI digest to release the tyrosinase from pBG130, then treating the 1.2 kb fragment with ExoIII to digest approximately 200 bases from the HindIII 3' end. After blunting the ends with T, DNA Polymerase, XhoI linkers were added and the 1.0 kb fragment was inserted into the Stratagene bluescript vector (Ks+; 2.9 kb) giving pBG132. The 1.0 kb tyrosinase gene was inserted into p803 (6.3 kb), which is a 3.6 kb subclone of the TMV plasmid, pS3-28 (available from W. O. Dawson, University of California, Riverside), in pUCl9, giving plasmids pBG21 and pBG22 (depending on the direction of the tyrosinase gene; 7.3 kb each). The plasmid S3-28 (11.1 kb) is a clone of TMV that has had the coat protein gene removed with an XhoI site at that position. Dawson, W. O. et al., Phytopathology 78, 783 (1988). A NcoI/EcoRV cut of the pBG21 and pBG22 released a 2.4 kb fragment containing the tyrosinase gene. This fragment replaced a 1.4 kb piece in pS3-28 that had been removed. The resulting plasmids, pBG23 and pBG24 (depending on the direction of the tyrosinase gene; 12.1 kb each) were the final DNA constructions that were used to make infectious RNA that were introduced into tobacco plants to introduce a mRNA for tyrosinase. This mRNA can be detected using northern blot procedures.

### EXAMPLE 2

### Preparation of Chimeric TMV DNA Comprising GUS Gene in Place of the Coat Protein Gene

The 1.8 kb GUS gene was derived from the pRAJ275 (4.5 kb; Clontech Laboratories) using a EcoRI/NcoI cut. The sticky ends were filled in using the Klenow fragment. XhoI linkers were added and the fragment was inserted into the Stratagene bluescript vector (KS+), which is 2.9 kb in size. The resulting plasmid was maintained as pBG150 (4.7 kb). Using the techniques of Example 1, this GUS gene with the XhoI linkers was moved into the p803, giving pBG25 and pBG26 (depending on the direction of the GUS gene; 8.1 kb each). A SalI/NcoI cut of the pBG25 and pBG26 released a 3.8 kb fragment containing the GUS gene. These fragments replaced a 2.0 kb piece in pS3-28 that had been removed. The resulting plasmids, pBG27 and pBG28 (depending on the direction of the GUS gene; 12.9 kb each) were the final DNA constructions that were used to make infectious RNA that were introduced into tobacco plants to introduce a mRNA for the GUS gene. This mRNA can be detected using northern blot procedures. The activity of the GUS enzyme is also detectable.

### EXAMPLE 3

### Preparation of Chimeric TMV DNA Comprising GUS/Coat Protein Gene in Place of the TMV Coat Protein Gene

The 1.8 kb GUS gene from pBG150 (4.7 kbk), described in Example 2 above, was used for this construction. A Pstl/NcoI cut of p35-5 released a 0.7 kb fragment that contains a portion of the 3' end and a portion of the 5' end of the TMV coat protein gene. The plasmid p35-5 (11.3 kb) is a clone of TMV that has had most of the coat protein gene removed and contains an XhoI site at the site where the internal portion of the coat protein gene is removed. Dawson, W. O. et al., Phytopathology 78, 783 (1988). This 0.7 kb fragment replaced a 0.5 kb piece in p803 that had been removed, giving pBG29 (6.5 kb). The 1.8 kb GUS gene was inserted into the XhoI site of pBG29, giving pBG31 and pBG32 (depending on the direction of the GUS/coat protein fusion gene; 8.3 kb each). A SalI/NcoI cut of the pBG31 and pBG32 released a 4.0 kb fragment containing the GUS/coat protein fusion gene. These fragments replaced a 2.0 kb piece in pS3-28 that had been removed. The resulting plasmids, pBG33 and pBG34 (depending on the direction of the GUS/coat protein fusion gene; 13.1 kb each) were the final DNA constructions that were used to make infectious RNA. pBG33, pBG34 and pS3-28 (as control) are transcribed into RNA which is used to infect tobacco plants by rubbing on the plants the RNA, an abrasive and a RNase inhibitor. Spots are noted on the plants indicating a successful infection. The infected plant tissue is macerated and fluorescence examined. Tissue infected with pS3-28 did not fluoresce whereas tissue infected with either pBG33 or pBG34 did fluoresce.

### EXAMPLE 4

### Preparation of a Non-Transmissible TMV Nucleotide Sequence

A full-length DNA copy of the TMV genome is prepared and inserted into the Pst I site of pBR322 as described by Dawson, W.O. et al., Proc. Nat. Acad. Sci. USA 83, 1832 (1986). The viral coat protein gene is located at position 5711 of the TMV genome adjacent the 30k protein gene. The vector containing the DNA copy of the TMV genome is digested with the appropriate restriction enzymes and exonucleases to delete the coat protein coding sequence. For example, the coat protein coding sequence is removed by a partial digestion with ClaI and NsiI, followed by relegation to reattach the 3'-tail of the virus. Alternatively, the vector is cut at the 3' end of the viral nucleic acid. The viral DNA is removed by digestion with Bal31 or exonuclease III up through the start codon of the coat protein coding sequence. A synthetic DNA sequence containing the sequence of the viral 3'-tail is then ligated to the remaining 5'-end. The deletion of the coding sequence for the viral coat protein is confirmed by isolating TMV RNA and using it to infect tobacco plants. The isolated TMV RNA is found to be non-infective, i.e. biologically contained, under natural conditions.

### EXAMPLE 5

### Preparation of a Non-Transmissible OMV Nucleotide Sequence

A full-length DNA copy of the OMV genome is prepared as described by Dawson, W.O. et al., (1986), supra. The vector containing the DNA copy of the OMV genome is digested with the appropriate restriction enzymes or suitable exonucleases such as described in Example 4 to delete the coat protein coding sequence. The deletion of the coding sequence for the viral coat protein is confirmed by isolating OMV RNA and using it to infect germinating barley plants. The isolated OMV RNA is found to be biologically contained under natural conditions.

### EXAMPLE 6 (not belonging to the invention)

### Preparation of a Non-Transmissible RNV Nucleotide Sequence

A full-length DNA copy of the RNV genome is prepared as described by Dawson, W.O. et al., (1986), supra. The vector containing the DNA copy of the RNV genome is digested with the appropriate restriction enzymes or suitable exonucleases such as described in Example 4 to delete the coat protein coding sequence. The deletion of the coding sequence for the viral coat protein is confirmed by isolating RNV RNA and using it to infect germinating barley plants. The isolated RNV RNA is found to be non-infective under natural conditions

### EXAMPLE 7 (not belonging to the invention)

### Preparation of a Non-Transmissible PVY or PVX Nucleotide Sequence

A full-length DNA copy of the PVY or PVX genome is prepared as described by Dawson, W.O. et al., (1986), supra. The vector containing the DNA copy of the PVY or PVX genome is digested with the appropriate restriction enzymes or suitable exonucleases such as described in Example 4 to delete the coat protein coding sequence. The deletion of the coding sequence for the viral coat protein is confirmed by isolating PVY or PVX RNA and using it to infect potato plants. The isolated PVY or PVX RNA is found to be biologically contained under natural conditions.

### EXAMPLE 8

### Preparation of Chimeric Nucleotide Sequence Containing the Tyrosinase Coding Sequence

The coding sequence for tyrosinase is isolated from Streptococcus antibioticus, by digestion with BclI followed by 5'-exonuclease digestion to the start codon. Alternatively, a restriction site is engineered adjacent the start codon of the tyrosinase coding sequence by site-directed oligonucleotide mutagenesis. Digestion with the appropriate restriction enzyme yields the coding sequence for tyrosinase. The fragment containing the tyrosinase coding sequence is isolated and cloned adjacent the promoter of the viral coat protein gene in the vectors prepared in Examples 4, 5 and 6.

### EXAMPLE 9 (not belonging to the invention)

### Preparation of Virus Containing Tyrosinase

A lambda P_{R} promoter is attached to the chimeric nucleotide sequence of Example 8 in accordance with the technique described in Dawson, W.O. et al., (186), supra. The resulting vector is used to transform E. coli, the production cell in this instance.

A second vector is prepared by inserting the viral coat protein coding sequence, isolated in Examples 4, 5 or 6, adjacent the lac promoter in the vector pBR322. This vector is used to transform the production cells having a vector with the corresponding compatible chimeric nucleotide sequence. The production cells are grown and the resultant viruses are isolated. Alternatively, the second vector is used to transform a second strain of E. coli which produces the coat protein. The coat protein and viral vector are then combined to form the virus.

### EXAMPLE 10 (not belonging to the invention)

### Production of Melanin

The viruses isolated in Example 9 are used to infect tobacco plants (viruses based on TMV) or germinating barley plants (viruses based on OMV or RNV). The infected plants are grown under normal growth conditions. The plants produce tyrosinase which reacts with' the components present in the plant tissue to produce intermediates which are converted to melanin within the plant tissue or after the plant tissue is lyzed. The melanin is isolated by conventional techniques.

### EXAMPLE 11

### Preparation of a Chimeric Nucleotide Sequence Containing a Beta Cyclodextrin Glucanotransferase Coding Sequence

The coding sequence for beta cyclodextrin glucotransferase is isolated from alkalophilic Bacillus sp. strain No. 38-2 in the following manner.

The chromosomal DNA of strain No. 38-2 (Hanamoto, T. et al., Agric. Biol. Chem. 51, 2019 (1987)) is partially cleaved with Sau3AI and the fragments are ligated in BamHI-digested pBR322. A transformant carrying plasmid pCS115, which contains a 3.2 kb DNA fragment from the genome of the producing strain, has the CGT activity. The CGT produced by this transformant gives one line of precipitation which fuses completely with that for the No. 38-2 CGT by an Ouchterlony double-diffusion test. The nucleotide sequence of the fragment is found by the dideoxy chain termination reaction (Sanger, F. et al., Proc. Nat. Acad. Sci. USA 74, 5463 (1977)) using pUCl9, and the exonuclease deletion method (Henikoff, S., Gene 28, 351 (1984)). The nucleotide sequence of the fragment shows a single open reading frame corresponding to the CGT gene. A protein with a molecular mass of 66 kDal could be translated from this open reading frame of 1758 bp. For the detailed nucleotide sequence, see Hanamoto, T. et al., supra.

The sequence of the N-terminal amino acids of the extracellular form of CGT is found with a peptide sequencer. NH₂-Ala-Pro-Asp-Thr-Ser-Val-Ser-Asn-Lys-Gln-Asn-Phe-Ser-Thr-Asp-Val-Ile is identical to that deduced from the DNA sequence (residues 1 to 17). This result suggests that 27 amino acid residues (residues -27 to - 1) represent a signal peptide which is removed during secretion of CGT. The molecular weight of the matured CGT calculated from the DNA sequence is 63,318.

A probe is prepared based on a portion of the amino acid sequence of cyclodextrin glucanotransferase and used to isolate the coding sequence for this enzyme. Alternatively, the beta cyclodextrin glucotransferase coding sequence is isolated following reverse transcription. The fragment containing the coding sequence is isolated and cloned adjacent the promoter of the viral coat protein gene in the vectors prepared in Examples 5, 6 or 7.

### EXAMPLE 12 (not belonging to the invention)

### Preparation of Virus Containing a Cyclodextrin Glucanotransferase Coding Sequence

A lambda P_{R} promoter is attached to the chimeric nucleotide sequence of Example 8 in accordance with the technique described in Dawson, W.O. et al., (1986), supra. The resulting vector is used to transform E. coli, the production cell in this instance.

A second vector is prepared by inserting the viral coat protein coding sequence, isolated in Examples 5, 6 or 7, adjacent the lac promoter in the vector pBR322. This vector is used to transform the production cells having a vector with the corresponding compatible chimeric nucleotide sequence. The production cells are grown and the resultant viruses are isolated.

### EXAMPLE 13 (not belonging to the invention)

### Production of Cyclodextrin

The viruses isolated in Example 12 are used to infect corn plants (viruses based on OMV or RNV) or potato plants (viruses based on PVY or PVX). The infected plants are grown under normal growth conditions. The plants produce cyclodextrin glucotransferase which catalyzes the conversion of starch to cyclodextrin in the plant tissue. The cyclodextrin is isolated by conventional techniques.

### EXAMPLE 14

### Preparation of a Chimeric Nucleotide Sequence of TMV and Chloramphenicol Acetyltransferase

A non-transmissible TMV nucleotide sequence (pTMVS3-28) is prepared as described in Example 4. A chimeric nucleotide sequence containing the chloramphenicol acetyltransferase (CAT) gene substituted for the previously removed coat protein gene (S3-CAT-28) is then prepared.

The CAT gene is removed from pCMl (Pharmacia) with Sal I and ligated into the nucleotide sequence from Example 4 which has been cleaved by Xho I. This construction produces pTMVS3-CAT-28 from which the mutant cp S3-CAT-28 is transcribed. Correct sequence and orientation is confirmed by sequencing as described by Sagursky, R.J. et al., Gene Anal. Technol. 2, 89 (1985).

This chimeric DNA sequence is then transcribed using the PM promoter (Ahlquist, P. et al., Mol. Cell Biol. 4, 2876 (1984)) and RNA polymerase of E. coli (Dawson, W.O. et al., Proc. Nat. Acad. Sci. USA, 83, 1832 (1986)) to produce infectious chimeric RNA. The infectivity is assayed by grinding leaves in cold 1% sodium pyrophosphate buffer, pH9.0, plus 1% bentonite and 1% Celite with immediate inoculation. Alternatively, the infectivity may be assayed using phenol-extracted RNA in the same buffer (Dawson, W.O., Virology, 73, 319 (1976)).

The chimeric RNA is found to replicate effectively in tobacco leaves. The RNA can be propagated serially from plant to plant or may be stored after phenol extraction. However, no virions are produced in the infected plants.

The chimeric RNA fails to produce a band during SDS-PAGE that can be positively identified as chloramphenicol acetyltransferase. However, functional enzyme activity can be detected in plant extracts that acetylated ¹⁴C-chloramphenicol with acetyl CoA.

### EXAMPLE 15 (not belonging to the invention)

### Preparation of a Virus Containing Chloramphenicol Acetyltransferase

A lambda P_{R} promoter is attached to the chimeric nucleotide sequence of Example 14 in accordance with the technique described by Dawson, W. o. et al., Proc. Nat. Acad. Sci. U.S.A. 83, 1832 (1986). The resulting vector is used to transform the production cell, E. coli

A second vector is prepared by inserting the viral coat protein coding sequence (isolated in Example 4), adjacent the lac promoter in the vector pBR322. This vector is used to transform the E. coli production cells having the first vector with the chimeric nucleotide sequence. The production cells are grown, and the resultant viruses are isolated. Alternatively, the second vector is used to transform a second strain of E. coli which produces the coat protein. The coat protein and viral vectors are then combined to form the virus.

### EXAMPLE 16 (not belonging to the invention)

### Production of Chloramphenicol Acetyltransferase

The viruses isolated in Example 15 are used to infect tobacco plants. The infected plants are grown under normal growth conditions. The plants produce the enzyme, chloramphenicol acetyltransferase, which is isolated from the plants using conventional techniques.

### EXAMPLE 17

### Transformation of Nicotiana Tobacum with Capsid Protein Gene

The capsid protein of tobacco mosaic virus (TMV) is encoded by a nucleotide sequence between 5712 and 6190. A 3'-untranslated region of the RNA genome extends to nucleotide 6395. A double stranded (ds) complementary DNA (cDNA) of the cistron containing nucleotide 5701 to 6395 and coding for capsid protein, is generated from TMV-RNA using reverse transcriptase. The transcriptase contains an oligonucleotide primer which is complementary to an oligonucleotide sequence at the 3' end of the viral RNA and contains a BamHI site.

A Klenow fragment of DNA polymerase is used to synthesize the second DNA-strand. The HindIII-BamHI fragment of cDNA is cloned into the plasmid pUC9 (Vieira, J. et al., Gene 19, 259 (1982)). The resulting plasmid is digested with AlaIII at nucleotide 5707 (Goelet, P. et al., Proc. Natl. Acad. Sci. USA 79, 5818 (1982)) and with BamHI to remove the sequence for capsid protein. This fragment is ligated into the vector pMON237.

pMON237 is a derivative of pMON200 (Horsch, R.B. et al., Science 227, 1229 (1985)). This vector contains a 19S CaMV promoter, a polylinker sequence and the sequence for nopaline synthese (NOS) at the 3' end. Ligation occurs at the XbaI site which is made blunt, and at the BamHI site. The capsid protein coding sequence is removed from the plasmid by digestion with XbaI and BamHI and given a BglII site at the 5' end and an EcoRI site at the 3' end by transfer to a suitable plasmid.

The BglII-EcoRI fragment is transferred into the expression cassette vector pMON316 between the CaMV 35S promoter and the NOS 3' untranslated region (Rogers, S.G. et al., in BioTechnology in Plant Science: Relevance to Agriculture in the Nineteen Eighties, M. Zaitlin, P. Day, A. Hollaender, Eds., Academic Press, N.Y., p. 219 (1986). The resulting chimeric gene contains the 35S promoter from CaMV and a polyadenylation signal from the NOS gene. The resulting plasmid is mated into Agrobacterium tumefaciens strain GV 3111 carrying the disarmed pTi BGS3-SE plasmid (Fraley, R.T. et al., Bio/Technology 3, 629 (1985)). A cointegrate plasmid results by recombination between LIH regions.

Transformed A. tumefaciens are selected for antibiotic resistance. Selected colonies are used to transform N. tobacum leaf disks which are then regenerated into whole plants (Horsch, R.B. et al., supra; Abel, P.P. et al., Science 232, 738 (1986)).
A. tumefaciens transformed with the sequence for TMV capsid protein is used as a production cell. Likewise tobacco cells that have been transformed with the sequence for TMV capsid protein are used as production cells for producing capsid protein monomers or multimers. Capsid protein numbers and timing of production can be controlled by methods well known in the arts (Maniatis, T. et al., supra). Proper selection of a temperature sensitive repressor and a strong promoter results in high copy numbers of plasmids and high translation rates only when desired.

### EXAMPLE 18

### Production of Encapsidated Virus Particles

Protoplasts of transformed tobacco cells obtained in the Example 49 are resuspended in a 0.5 M mannitol solution containing 12-30 nM MgCl₂. A heat shock of 45°C for five minutes is given. The protoplasts are distributed in aliquots for transformation in centrifuge tubes, 0.3 ml of suspended protoplasts per tube. During the next 10 minutes the following are added. Plasmids obtained in Example 1 containing cDNA coding for the TMV genome but missing the gene for capsid protein, and which contains a chimeric gene construct; and polyethylene glycol (PEG) solution (MW 6000, 40% (w/v) containing 0.1M Ca(NO₃)₂ and 0.4M mannitol; pH 8-9 with KOH) to give a final concentration of 20% PEG. The aliquots are incubated for 30 minutes with occasional gentle shaking, and then the protoplasts are placed in petri dishes (0.3 ml original protoplast suspension per 6 cm diameter dish) and cultured. Encapsidated virus particles are isolated from the protoplasts using conventional techniques.

Alternatively, the transformed plants produced in Example 49 are infected with virus nucleic acid, e.g. a chimeric nucleotide sequence of Example 3. The nucleic acid is replicated and coat protein is produced which encapsidates the nucleic acid. The encapsidated virus particles are isolated using conventional techniques.

### EXAMPLE 19

### Transformation of Nicotiana Tobacum with Replicase Gene

Two replicase subunits of tobacco mosaic virus (TMV) are encoded by a nucleotide sequence between 70 and 4919. The nucleotide sequence for the 130-kD protein terminates at an amber codon which can be suppressed to produce a 180-kD protein. A double stranded (ds) complementary DNA (cDNA) of the cistron containing nucleotides 70 to 4919 and coding for replicase, is generated from TMV-RNA using reverse transcriptase.

The transcriptase contains an oligonucleotide primer which is complementary to an oligonucleotide sequence at the 3' end of the viral RNA and contains a BamHI site. A Klenow fragment of DNA polymerase is used to synthesize the second DNA-strand. The HindIII-BamHi fragment of cDNA is cloned into the plasmid pUC9 (Viera, J. et al., Gene 19, 259 (1982)). The resulting plasmid is digested with AlaIII at nucleotide 4919 (Goelet, P. et al., Proc. Natl. Acad. Sci. USA 79, 5818 (1982)) and with BamHI to remove the sequence for capsid protein.

This fragment is ligated into the vector pMON237. pMON237 is a derivative of pMON200 (Horsch, R.B. et al., Science 227, 1229 (1985)). This vector contains a 19S CaMV promoter, a polylinker sequence and the sequence for nopaline synthase (NOS) at the 3' end. Ligation occurs at the XbaI site which is made blunt, and at the BamHI site. The capsid protein coding sequence is removed from the plasmid by digestion with XbaI and BamHI and given a BglII site at the 5' end and EcoRI site at the 3' end by transfer to a suitable plasmid. The BglII-EcoRI fragment is transferred into the expression cassette vector, pMON316 between the CaMV 35S promoter and the NOS 3' untranslated region (Rogers, S.G. et al., in BioTechnology in Plant Science: Relevance to Agriculture in the Nineteen Eighties, M. Zaitlin, P. Day, A. Hollaender, Eds., Academic Press, N.Y., p. 219, 1986). The resulting chimeric gene contains the 35S promoter from CaMV and a polyadenylation signal from the NOS gene. The resulting plasmid is mated into Agrobacterium tumefaciens strain GV 3111 carrying the disarmed pTi BGS3-SE plasmid (Fraley, R.T. et al., Bio Technology 3, 629 (1985)). A cointegrate plasmid results by recombination between LIH regions. Transformed A. tumefaciens are selected for antibiotic resistance. Selected colonies are used to transform N. tobacum leaf disks which are then regenerated into whole plants (Horsch, R.B. et al., supra, Abel, P.P. et al., Science 232, 738 (1986)). The transformed hosts can be used as production cells or hosts for the infection with TMV based viruses. The stably incorporated replicase will function to enhance replication of the viral nucleic acid.

### EXAMPLE 20

### Transformation of Tobacco Cells with Transport Protein

A nucleotide sequence of TMV from nucleotide 4903 to 5709 encodes a 30-kD protein which is implicated in facilitating cell to cell movement of the virus. Leaf disk cells of N. tobacum are transformed by Agrobacterium carrying a cointegrate plasmid made by the method of Example 49. N. tobacum cells so transformed are used as host cells for encapsidated defective virus particles.

## Claims

1. A production cell that produces a transmissible and infective virus comprising:
(1) a first vector which comprises a chimeric nucleotide sequence comprising:
(a) a first nucleotide sequence having substantial sequence homology to a plant viral nucleotide sequence derived from a tobamovirus which is capable of replication and is biologically contained, wherein said viral nucleotide sequence lacks a biologically functional viral coat protein coding sequence; and
(b) a second nucleotide sequence which is adjacent to a viral promoter and which is a coding sequence of interest capable of transcription in the production cell; and
(2) a third nucleotide sequence coding for a biologically functional viral coat protein wherein the viral coat protein is compatible with the first nucleotide sequence and wherein the third nucleotide sequence is stably incorporated in the production cell genome.

2. The production cell of claim 1, wherein the nucleotide sequence of interest codes for the expression of one or more phenotypic traits.

3. The production cell of claim 2, wherein the phenotypic traits include improved tolerance to herbicides; improved tolerance to extremes of heat or cold, drought, salinity or osmotic stress; improved resistance to pests or diseases; production of enzymes or secondary metabolites; male or female sterility; dwarfness; early maturity; improved yield, vigor, heterosis, nutritional qualities, flavor or processing properties; production of important proteins or products for commercial use; production of degradative or inhibitory enzymes.

4. The production cell of any of claims 1 to 3, wherein the viral nucleotide sequence further lacks a coding sequence for a biologically functional viral transmissibility factor.

5. The production cell of any of claims 1 to 4, wherein the viral promoter is a viral coat protein promoter.

6. A process of producing a product in a plant cell comprising:
(a) infecting a plant cell with the first vector as defined in any of claims 1 to 5, wherein the plant cell has been transformed in advance such that the coding sequence for the biologically functional viral coat protein is stably incorporated into the genome of the plant cell;
(b) growing the infected plant cell to produce the product of the second nucleotide sequence contained in the first vector; and
(c) isolating the product of the second nucleotide sequence,
wherein the plant cell is part of a whole plant.

7. The process of claim 6, wherein said product is an anti-sense RNA, a ribozyme, a protein, an enzyme or a complex biomolecule.

8. The process of claim 7, wherein the biomolecule is melanin or cyclodextrin.

9. The process of claim 7, wherein the enzyme is tyrosinase, cylodextrin gluconotransferase or human tissue plasminogen activator.

10. A plant cell which has been infected with the first vector as defined in any of claims 1 to 5, wherein the plant cell has been transformed in advance such that the coding sequence for the biologically functional viral coat protein is stably incorporated into the genome of the plant cell, and wherein the plant cell is part of a whole plant.

11. The plant cell of claim 10, wherein the nucleotide sequence of interest codes for the expression of one or more phenotypic traits.

12. The plant cell of claim 11, wherein the phenotypic traits include improved tolerance to herbicides; improved tolerance to extremes of heat or cold, drought, salinity or osmotic stress; improved resistance to pests or diseases; production of enzymes or secondary metabolites; male or female sterility; dwarfness; early maturity; improved yield, vigor, heterosis, nutritional qualities, flavor or processing properties; production of important proteins or products for commercial use; production of degradative or inhibitory enzymes.

13. The plant cell of any of claims 10 to 12, wherein the viral nucleotide sequence further lacks a coding sequence for a biologically functional viral transmissibility factor.

14. The plant cell of any of claims 10 to 13, wherein the viral promoter is a viral coat protein promoter.

## Patentansprüche

1. Produktionszelle, die ein übertragbares und infektiöses Virus produziert, die umfasst:
(1) einen ersten Vektor, der eine chimäre Nukleotidsequenz aufweist, welche umfasst:
(a) eine erste Nukleotidsequenz, die eine substantielle Sequenzhomologie mit der Nukleotidsequenz eines Pflanzenvirus aufweist, welche von einem Tobamovirus abstammt, die zur Replikation imstande ist und biologisch eingedämmt ist, wobei der viralen Nukleotidsequenz eine kodierende Sequenz für ein biologisch funktionelles virales Hüllprotein fehlt; und
(b) eine zweite Nukleotidsequenz, die an einen viralen Promotor angrenzt, und die eine kodierende Sequenz von Interesse darstellt, welche zur Transkription in der Produktionszelle imstande ist; und
(2) eine dritte Nukleotidsequenz, die für ein biologisch funktionelles virales Hüllprotein kodiert, wobei das virale Hüllprotein mit der ersten Nukleotidsequenz kompatibel ist, und wobei die dritte Nukleotidsequenz stabil in das Genom der Produktionszelle integriert ist.

2. Produktionszelle nach Anspruch 1, wobei die Nukleotidsequenz von Interesse für die Expression von einem oder mehr phänotypischen Merkmalen kodiert.

3. Produktionszelle nach Anspruch 2, wobei die phänotypischen Merkmale verbesserte Toleranz gegenüber Herbiziden, verbesserte Toleranz gegenüber extremer Hitze oder Kälte, Trockenheit, Salzhaltigkeit oder osmotischem Stress, verbesserte Resistenz gegenüber Pflanzenschädlingen oder Krankheiten, Produktion von Enzymen oder sekundären Metaboliten, männliche oder weibliche Sterilität, Zwergwuchs, Frühreife, verbesserter Ertrag, Vitalität, Heterosis, Nährstoffqualität, Geschmack oder Verarbeitungseigenschaften, Produktion wichtiger Proteine oder von Produkten zur kommerziellen Verwendung und Produktion abbauender oder inhibitorischer Enzyme einschließen.

4. Produktionszelle nach einem der Ansprüche 1 bis 3, wobei der viralen Nukleotidsequenz zusätzlich eine kodierende Sequenz für einen funktionellen viralen Transmissionsfaktor fehlt.

5. Produktionszelle nach einem der Ansprüche 1 bis 4, wobei der virale Promotor ein Promotor für ein virales Hüllprotein ist.

6. Verfahren zur Herstellung eines Produkts in einer Pflanzenzelle, das umfasst:
(a) Infizieren einer Pflanzenzelle mit dem ersten Vektor wie in einem der Ansprüche 1 bis 5 definiert, wobei die Pflanzenzelle zuvor dergestalt transformiert wurde, dass die kodierende Sequenz für das biologisch funktionelle virale Hüllprotein stabil in das Genom der Pflanzenzelle integriert ist;
(b) Anzucht der infizierten Pflanzenzelle, um das Produkt der zweiten Nukleotidsequenz zu produzieren, welche im ersten Vektor enthalten ist; und
(c) Isolieren des Produkts der zweiten Nukleotidsequenz,
wobei die Pflanzenzelle Teil einer ganzen Pflanze ist.

7. Verfahren nach Anspruch 6, wobei das Produkt eine antisense RNA, ein Ribozym, ein Protein, ein Enzym oder ein komplexes Biomolekül ist.

8. Verfahren nach Anspruch 7, wobei das Biomolekül Melanin oder Cyclodextrin ist.

9. Verfahren nach Anspruch 7, wobei das Enzym Tyrosinase, Cyclodextrin-Gluconotransferase oder humaner Gewebe-Plasminogenaktivator ist.

10. Pflanzenzelle, die mit dem ersten Vektor wie in einem der Ansprüche 1 bis 5 definiert infiziert wurde, wobei die Pflanzenzelle zuvor dergestalt transformiert wurde, dass die kodierende Sequenz für das biologisch funktionelle virale Hüllprotein stabil in das Genom der Pflanzenzelle integriert ist, und wobei die Pflanzenzelle Teil einer ganzen Pflanze ist.

11. Pflanzenzelle nach Anspruch 10, wobei die Nukleotidsequenz von Interesse für die Expression von einem oder mehr phänotypischen Merkmalen kodiert.

12. Pflanzenzelle nach Anspruch 11, wobei die phänotypischen Merkmale verbesserte Toleranz gegenüber Herbiziden, verbesserte Toleranz gegenüber extremer Hitze oder Kälte, Trockenheit, Salzhaltigkeit oder osmotischem Stress, verbesserte Resistenz gegenüber Pflanzenschädlingen oder Krankheiten, Produktion von Enzymen oder sekundären Metaboliten, männliche oder weibliche Sterilität, Zwergwuchs, Frühreife, verbesserter Ertrag, Vitalität, Heterosis, Nährstoffqualität, Geschmack oder Verarbeitungseigenschaften, Produktion wichtiger Proteine oder von Produkten zur kommerziellen Verwendung und Produktion abbauender oder inhibitorischer Enzyme einschließen.

13. Pflanzenzelle nach einem der Ansprüche 10 bis 12, wobei viralen Nukleotidsequenz zusätzlich eine kodierende Sequenz für einen funktionellen viralen Transmissionsfaktor fehlt.

14. Pflanzenzelle nach einem der Ansprüche 10 bis 13, wobei der virale Promotor ein Promotor für ein virales Hüllprotein ist.

## Revendications

1. Cellule de production qui produit un virus transmissible et infectieux, comprenant :
(1) un premier vecteur qui comprend une séquence nucléotidique chimérique comprenant :
(a) une première séquence nucléotidique ayant une homologie de séquence substantielle avec une séquence nucléotidique d'un virus de plante, dérivée d'un tobamovirus, qui est capable de réplication et qui est biologiquement confiné, ladite séquence nucléotidique virale étant dépourvue de séquence codant une protéine d'enveloppe virale biologiquement fonctionnelle ; et
(b) une seconde séquence nucléotidique qui est adjacente à un promoteur viral et qui est une séquence codante d'intérêt capable de transcription dans la cellule de production ; et
(2) une troisième séquence nucléotidique codant une protéine d'enveloppe virale biologiquement fonctionnelle, la protéine d'enveloppe virale étant compatible avec la première séquence nucléotidique, et la troisième séquence nucléotidique étant incorporée de façon stable dans le génome de la cellule de production.

2. Cellule de production selon la revendication 1, dans laquelle la séquence nucléotidique d'intérêt code l'expression d'un ou plusieurs traits phénotypiques.

3. Cellule de production selon la revendication 2, dans laquelle les traits phénotypiques comprennent une tolérance améliorée à des herbicides ; une tolérance améliorée à des extrêmes de chaleur ou de froid, de sécheresse, de salinité ou de stress osmotique ; une résistance améliorée à des pesticides ou à des maladies ; la production d'enzymes ou de métabolites secondaires ; une stérilité mâle ou femelle ; un nanisme ; une maturité précoce ; un rendement, une vigueur, une hétérosis, des qualités nutritionnelles, un arôme ou des propriétés de traitement améliorés ; la production de protéines ou de produits importants pour une utilisation commerciale ; la production d'enzymes de dégradation ou inhibitrices.

4. Cellule de production selon l'une quelconque des revendications 1 à 3, dans laquelle la séquence nucléotidique virale est en outre dépourvue d'une séquence codant un facteur de transmissibilité viral biologiquement fonctionnel.

5. Cellule de production selon l'une quelconque des revendications 1 à 4, dans laquelle le promoteur viral est un promoteur de protéine d'enveloppe virale.

6. Procédé de production d'un produit dans une cellule végétale, comprenant :
(a) le fait d'infecter une cellule végétale avec le premier vecteur tel qu'il est défini dans l'une quelconque des revendications 1 à 5, la cellule végétale ayant été transformée à l'avance de sorte que la séquence codant la protéine d'enveloppe virale biologiquement fonctionnelle soit incorporée de façon stable dans le génome de la cellule végétale ;
(b) le fait de faire croître la cellule végétale infectée pour produire le produit de la seconde séquence nucléotidique contenue dans le premier vecteur ; et
(c) le fait d'isoler le produit de la seconde séquence nucléotidique,
où la cellule végétale est une partie d'une plante entière.

7. Procédé selon la revendication 6, dans lequel ledit produit est un ARN anti-sens, un ribozyme, une protéine, une enzyme ou une molécule biologique complexe.

8. Procédé selon la revendication 7, dans lequel la molécule biologique est la mélanine ou la cyclodextrine.

9. Procédé selon la revendication 7, dans lequel l'enzyme est une tyrosinase, une cyclodextrine glucanotransférase ou un activateur tissulaire du plasminogène humain.

10. Cellule végétale qui a été infectée par le premier vecteur tel qu'il est défini dans l'une quelconque des revendications 1 à 5, la cellule végétale ayant été transformée à l'avance de sorte que la séquence codant la protéine d'enveloppe virale biologiquement fonctionnelle soit incorporée de façon stable dans le génome de la cellule végétale, et la cellule végétale étant une partie d'une plante entière.

11. Cellule végétale selon la revendication 10, dans laquelle la séquence nucléotidique d'intérêt code l'expression d'un ou plusieurs traits phénotypique.

12. Cellule végétale selon la revendication 11, dans laquelle les traits phénotypiques comprennent une tolérance améliorée à des herbicides ; une tolérance améliorée à des extrêmes de chaleur ou de froid, de sécheresse, de salinité ou de stress osmotique ; une résistance améliorée à des pesticides ou à des maladies ; la production d'enzymes ou de métabolites secondaires ; une stérilité mâle ou femelle ; un nanisme ; une maturité précoce ; un rendement, une vigueur, une hétérosis, des qualités nutritionnelles, un arôme ou des propriétés de traitement améliorés ; la production de protéines ou de produits importants pour une utilisation commerciale ; la production d'enzymes de dégradation ou inhibitrices.

13. Cellule végétale selon l'une quelconque des revendications 10 à 12, dans laquelle la séquence nucléotidique virale est en outre dépourvue d'une séquence codant un facteur de transmissibilité viral biologiquement fonctionnel.

14. Cellule végétale selon l'une quelconque des revendications 10 à 13, dans laquelle le promoteur viral est un promoteur de protéine d'enveloppe virale.
